# FASCICULE DE BREVET EUROPEEN

(11) **EP 0 580 514 B1**
(45) Date de publication et mention de la délivrance du brevet: **25.09.1996**
(21) Numéro de dépôt: 93401901.9
(22) Date de dépôt: 22.07.1993
(51) Int. Cl.: A61K 7/06

(54) **Laque aérosol capillaire à base de N-carboxybutyl chitosane**
Aerosol-Haarspray mit N-Carboxybutyl-Chitosan
Aerosol hair lacqueur based on N-carboxybutyl chitosan

(30) Priorité: 24.07.1992 FR 9209177
(43) Date de publication de la demande: 26.01.1994
(73) Titulaire: L'OREAL, F-75008 Paris (FR)
(72) Inventeur: Dupuis, Christine, F-75018 Paris (FR); Dubief, Claude, F-78150 Le Chesnay (FR)
(74) Mandataire: Stalla-Bourdillon, Bernard

(56) Documents cités:
- GB-A- 2 189 145
- CARBOHYDRATE POLYMERS vol. 11, no. 4, 1989, pages 307 - 320 R. MUZZARELLI ET EL. 'Characteristic Properties of N-Carboxybutyl Chitosan' Page 316, ü1; page 317, last ü - page 319, ü2

## Description

La présente invention a pour objet une laque aérosol capillaire pour le maintien et/ou la fixation de la chevelure contenant en tant que substance filmogène du N-carboxybutyl chitosane.

Les laques aérosols capillaires sont essentiellement constituées d'une solution aqueuse ou hydroalcoolique, d'une substance filmogène en mélange éventuellement avec divers adjuvants cosmétiques tels que des agents plastifiants, des agents adoucissants, des parfums, des huiles, des lubrifiants, de la lanoline, des silicones, des filtres solaires ou des colorants. Cette solution est ensuite conditionnée dans un récipient aérosol approprié qui est mis sous pression à l'aide d'un gaz propulseur. Jusqu'à ces derniers temps, les gaz propulseurs étaient essentiellement des chlorofluoroalcanes tels que les produits vendus sous les dénominations de "Fréon".

Pour des raisons essentiellement écologiques, les agents propulseurs du type chlorofluoroalcane sont à l'heure actuelle le plus souvent remplacés par des agents propulseurs présentant moins de risques pour la couche d'ozone tels que le diméthyléther, les hydrocarbures volatils comme le propane, le n-butane, l'isobutane ou leurs mélanges et les hydrochlorofluoroalcanes ou hydrofluoroalcanes ou un mélange de ces agents propulseurs.

Pour d'autres raisons, on a également cherché à remplacer totalement ou partiellement le solvant de la substance filmogène par de l'eau.

Ces modifications portant à la fois sur la nature de l'agent propulseur et du solvant de la substance filmogène ont entraîné des phénomènes d'incompatibilité, en particulier de précipitation de la substance filmogène ce qui, par voie de conséquence, a conduit à une obturation des valves.

En vue d'obtenir une bonne laque aérosol ne présentant pas de phénomènes de précipitation, tout en ayant une pulvérisation fine et un bon pouvoir laquant, il convenait donc de choisir une substance filmogène appropriée présentant non seulement une bonne solubilité dans le solvant mais également une bonne compatibilité avec l'agent propulseur.

Dans cette optique, les recherches se sont portées sur les N-carboxyalkyl chitosanes notamment le N-carboxyméthyl chitosane et le N-carboxybutyl chitosane qui ont été décrits dans certaines applications cosmétiques, en particulier dans des crèmes, laits et gels, comme substituts de l'acide hyaluronique du fait de leur pouvoir hydratant élevé et protecteur de la peau vis-à-vis des agressions extérieures.

On peut à cet égard citer la demande de brevet japonais (Kokai) n° 63.230614 qui décrit une composition cosmétique contenant du carboxyméthyl chitosane et un ou plusieurs mucopolysaccharides, ainsi que la publication de R. Muzzarelli et ai., Chimica. Oggi., pp.33-37, Avril 1991.

MUZZARELLI et al. ont également décrit dans Carbohydrate Polymers, 11 (1989), 307-320, un certain nombre de propriétés des N-carboxybutyl chitosanes.

Par ailleurs, GB-2 189 145 décrit des compositions pour combattre l'aspect gras des cheveux contenant au moins un polymère dérivé du chitosane.

Les études effectuées sur ces deux dérivés hydrosolubles du chitosane ont montré qu'ils présentaient un comportement différent en fonction de la nature du solvant et surtout de celle de l'agent propulseur.

De façon tout à fait inattendue et surprenante, il s'est avéré que le N-carboxybutyl chitosane, contrairement au N-carboxyméthyl chitosane, était parfaitement compatible avec un agent propulseur bien déterminé, à savoir le diméthyléther. Par ailleurs, les laques aérosols réalisées à base de N-carboxybutyl chitosane, en tant que substance filmogène, et de diméthyléther, en tant qu'agent propulseur, se sont avérées présenter un excellent pouvoir laquant.

La présente invention a donc pour objet à titre de produit industriel nouveau une laque aérosol pour le maintien et/ou la fixation de la chevelure contenant, dans un récipient aérosol approprié, un véhicule aqueux cosmétiquement acceptable, du N-carboxybutyl chitosane en tant que substance filmogène et du diméthyléther en tant qu'agent propulseur.

Les N-carboxybutyl chitosanes sont des composés bien connus qui sont constitués dans la chaîne polysaccharidique d'unités de glucosamine, de N-acétylglucosamine et de N-carboxybutyl glucosamine.

Le poids moléculaire des N-carboxybutyl chitosanes est généralement très élevé et est de préférence compris entre 400.000 et 700.000 Daltons, déterminé par spectrométrie laser à diffraction de lumière sur des solutions diluées, filtrées à travers des membranes de 0,2 µm en présence de chlorure de sodium.

Les N-carboxybutyl chitosanes sont du type amphotère avec un caractère cationique prévalent.

Les N-carboxybutyl chitosanes peuvent être préparés selon le procédé décrit dans le brevet US 4.835.265 à partir de chitosane et d'acide lévulinique ou 4-oxo-pentanoïque.

Parmi les N-carboxybutyl chitosanes du commerce, on peut utiliser selon l'invention ceux vendus par la Société Jan Dekker International sous la dénomination de "Evalsan'® sous forme d'une solution à 1 % dans l'eau.

Des teneurs en N-carboxybutyl chitosane supérieures à 1 % peuvent être obtenues par lyophilisation.

Dans les laques aérosols selon l'invention, le N-carboxybutyl chitosane peut être présent à une concentration comprise entre 0,1 et 10 % en poids par rapport au poids total de la laque et de préférence entre 0,5 et 6 % en poids.

Le véhicule aqueux est soit de l'eau, soit un mélange d'eau et d'un solvant organique cosmétiquement acceptable tel qu'un monoalcool ayant de 1 à 8 atomes de carbone comme l'éthanol, l'isopropanol, l'alcool benzylique, l'alcool phényléthylique ou un polyalcool tel que les alkylène glycols comme l'éthylène glycol, le propylène glycol et les mélanges de ces solvants.

Lorsque le véhicule aqueux contient un solvant, celui-ci est de préférence présent en une proportion inférieure à 50 % en poids par rapport au poids total de la composition et de préférence, compris entre 5 et 30 %.

Selon une variante de l'invention, le N-carboxybutyl chitosane peut être associé à une substance filmogène secondaire choisie par exemple parmi :
- le copolymère vinylpyrrolidone/acétate de vinyle vendu sous la dénomination de "Luvisquol VA 64 Poudre"® par la Société BASF ou de "PVP/VA S630" par la Société GAF,
- le copolymère acétate de vinyle/acide crotonique (90/10) sous la dénomination de "Luviset CA 66"® vendu par la Société BASF, ou
- la polyvinylpyrrolidone vendue sous la dénomination de "Polyvinyl pyrrolidone K30'® par la Société GAF.

La solution aqueuse peut également contenir un agent plastifiant dans une proportion d'au moins 10 % en poids par rapport au poids des substances filmogènes et de préférence entre 20 et 50 %.

L'agent plastifiant, tout comme le N-carboxybutyl chitosane, doit être soluble dans le véhicule aqueux et les mélanges du véhicule aqueux et du diméthyléther. Par ailleurs, l'agent plastifiant doit être compatible avec le N-carboxybutyl chitosane, c'est-à-dire qu'il doit pouvoir s'insérer moléculairement dans le film de résine sans exuder.

La composition à pulvériser peut également contenir des additifs cosmétiques conventionnels compatibles avec le N-carboxybutyl chitosane tels que par exemple des agents adoucissants, des parfums, des silicones, des filtres solaires, des colorants, des conservateurs, des émulsifiants, des agents anti-mousse, des vitamines, des protéines, des hydratants ainsi que des agents anti-radicaux libres.

L'agent propulseur, à savoir le diméthyléther, est généralement présent en une proportion comprise entre 10 et 90 %, et de préférence entre 20 et 40 % en poids par rapport au poids total de la composition. Il est souhaitable que la pression de vapeur interne soit comprise entre 2.10⁵ et 5.10⁵ Pa.

Le diméthyléther peut éventuellement être associé à au moins un agent propulseur secondaire tel qu'un hydrochlorofluoroalcane, un hydrofluoroalcane, un hydrocarbure non halogéné comme le propane, le n-butane, l'isobutane ou leurs mélanges.

Lorsque la composition contient plus de 40 % en poids de diméthyléther, il est souhaitable, en vue de préserver l'homogénéité de la composition, d'y inclure un agent émulsifiant. Cet agent émulsifiant est de préférence du type non-ionique notamment un copolymère oxyde d'éthylène/oxyde de propylène et est présent à une concentration inférieure à 0,2 % en poids par rapport au poids total de la composition.

Le récipient aérosol dans lequel la composition est conditionnée est du type classique mais peut être éventuellement équipé d'une prise de gaz additionnel en vue d'obtenir une pulvérisation de qualité plus fine.

On va maintenant donner à titre d'illustration plusieurs exemples de laques aérosols selon l'invention.

### EXEMPLE 1 :

On prépare une laque aérosol pour cheveux en conditionnant, dans un récipient aérosol approprié, 0,68 g de N-carboxybutyl chitosane vendu en solution aqueuse à 1 % sous la dénomination de "Evalsan"® par la Société Jan Dekker, puis 2,1 g de copolymère vinylpyrrolidone/acétate de vinyle (65/35) vendu sous la dénomination de "PVP/VA S630® par la Société GAF ainsi qu'un parfum et un conservateur.

A la solution ainsi obtenue, on ajoute selon les techniques conventionnelles, 30 g de diméthyléther et on procède à la fixation de la valve et à la fermeture hermétique du récipient (pression : 4,7.10⁵ Pa à 20°C).

Par pulvérisation de la laque sur des cheveux secs préalablement coiffés, on obtient une excellente fixation de la chevelure dans le temps et une bonne élimination par simple brossage.

### EXEMPLE 2 :

Selon le même mode opératoire que celui décrit à l'exemple 1, on a préparé une laque aérosol capillaire ayant la composition suivante :

| | |
|---|---|
| - N-carboxybutyl chitosane vendu en solution aqueuse à 1 % sous la dénomination de "Evalsan'® par la Société Jan Dekker | 0,55 g(MA) |
| - Parfum, conservateur... qs | |
| - Copolymère d'oxyde d'éthylène/oxyde de propylène vendu sous la dénomination de "Pluronic 9400'® par la Société BASF-Wyandotte | 0,011 g |
| - Diméthyléther Pression 5.10⁵ Pa à 20°C. | 45 g |

### EXEMPLE 3 :

On prépare une laque aérosol capillaire en conditionnant dans un récipient aérosol approprié 0,59 g de N-carboxybutyl chitosane lyophilisé, 1,3 g de polyvinyl pyrrolidone en poudre vendu sous la dénomination de "Polyvinyl pyrrolidone K30'® par la Société GAF, 6,5 g d'alcool éthylique absolu ainsi qu'un parfum, un conservateur et la quantité d'eau suffisante pour compléter à 65g.

A la solution obtenue, on ajoute ensuite selon les techniques conventionnelles 35 g de diméthyléther et on procède à la fixation de la valve et à la fermeture hermétique du récipient (pression 4,7.10⁵ Pa à 20°C).

### EXEMPLE 4 :

Selon le même mode opératoire que celui décrit à l'exemple 3, on prépare une laque aérosol capillaire ayant la composition suivante :

| | |
|---|---|
| - N-carboxybutyl chitosane lyophilisé | 1 g |
| - Parfum, colorant, conservateur qs | |
| - Eau déminéralisée qsp | 100 g. |

A 70 g de la solution obtenue, on ajoute selon les techniques conventionnelles 30 g de diméthyléther et on procède à la fixation de la valve et à la fermeture hermétique du récipient (pression 4,7.10⁵ Pa à 20°C).

## Revendications

1. Laque aérosol pour le maintien et/ou la fixation de la chevelure, caractérisée par le fait qu'elle contient dans un récipient aérosol approprié, un véhicule aqueux cosmétiquement acceptable, du N-carboxybutyl chitosane en tant que substance filmogène et du diméthyléther en tant qu'agent propulseur.

2. Laque aérosol selon la revendication 1, caractérisée par le fait que le N-carboxybutyl chitosane est présent à une concentration comprise entre 0,1 et 10 % en poids, par rapport au poids total de la laque.

3. Laque aérosol selon la revendication 1 ou 2, caractérisée par le fait que le véhicule aqueux est de l'eau ou un mélange d'eau et d'un solvant organique, cosmétiquement acceptable.

4. Laque aérosol selon la revendication 3, caractérisée par le fait que le solvant organique cosmétiquement acceptable est l'éthanol, l'isopropanol, l'alcool benzylique, l'alcool phényléthylique, l'éthylèneglycol, le propylèneglycol ou un mélange de ces solvants.

5. Laque aérosol selon la revendication 3 ou 4, caractérisée par le fait que le solvant est présent en une proportion inférieure à 50 % et de préférence comprise entre 5 et 30 % en poids par rapport au poids total de la composition.

6. Laque aérosol selon l'une quelconque des revendications précédentes, caractérisée par le fait qu'elle contient une substance filmogène secondaire.

7. Laque aérosol selon l'une quelconque des revendications précédentes, caractérisée par le fait qu'elle contient en outre un agent plastifiant en une proportion d'au moins 10 % en poids par rapport au poids total des substances filmogènes.

8. Laque aérosol selon l'une quelconque des revendications précédentes, caractérisée par le fait que le diméthyléther est présent en une proportion comprise entre 10 et 90 % et de préférence entre 20 et 40 % en poids par rapport au poids total de la composition.

9. Laque aérosol selon l'une quelconque des revendications précédentes, caractérisée par le fait qu'elle contient un agent propulseur secondaire choisi parmi les hydrochlorofluoroalcanes, les hydrofluoroalcanes et les hydrocarbures non-halogénés et leurs mélanges.

10. Laque aérosol selon l'une quelconque des revendications précédentes, caractérisée par le fait qu'elle contient en outre un agent adoucissant, un parfum, une silicone, un filtre solaire, un colorant, un conservateur, un émulsifiant, un agent anti-mousse, une vitamine, une protéine, un hydratant, un agent anti-radicaux libres.

## Claims

1. Aerosol lacquer for supporting and/or fixing hair, characterized in that it contains, in a suitable aerosol receptacle, a cosmetically acceptable aqueous carrier, N-carboxybutylchitosan as film-forming substance and dimethyl ether as propellant.

2. Aerosol lacquer according to Claim 1, characterized in that N-carboxybutylchitosan is present in a concentration of between 0.1 and 10 % by weight, relative to the total weight of the lacquer.

3. Aerosol lacquer according to Claim 1 or 2, characterized in that the aqueous carrier is water or a mixture of water and of a cosmetically acceptable organic solvent.

4. Aerosol lacquer according to Claim 3, characterized in that the cosmetically acceptable organic solvent is ethanol, isopropanol, benzyl alcohol, phenethyl alcohol, ethylene glycol, propylene glycol or a mixture of these solvents.

5. Aerosol lacquer according to Claim 3 or 4, characterized in that the solvent is present in a proportion which is lower than 50 % and preferably between 5 and 30 % by weight relative to the total weight of the composition.

6. Aerosol lacquer according to any one of the preceding claims, characterized in that it contains a secondary film-forming substance.

7. Aerosol lacquer according to any one of the preceding claims, characterized in that it additionally contains a plasticizing agent in a proportion of at least 10 % by weight relative to the total weight of the film-forming substances.

8. Aerosol lacquer according to any one of the preceding claims, characterized in that dimethyl ether is present in a proportion of between 10 and 90 % and preferably between 20 and 40 % by weight relative to the total weight of the composition.

9. Aerosol lacquer according to any one of the preceding claims, characterized in that it contains a secondary propellant chosen from hydrochlorofluoroalkanes, hydrofluoroalkanes and unhalogenated hydrocarbons and their mixtures.

10. Aerosol lacquer according to any one of the preceding claims, characterized in that it additionally contains an emollient, a perfume, a silicone, a sunscreen, a colorant, a preserving agent, an emulsifier, an antifoam agent, a vitamin, a protein, a hydrating agent and an agent against free radicals.

## Patentansprüche

1. Aerosollack zum Festigen und/oder Fixieren des Haars, dadurch gekennzeichnet, daß er in einem geeigneten Aerosolbehälter einen kosmetisch annehmbaren wäßrigen Träger aus N-Carboxybutylchitosan als filmbildende Substanz und Dimethylether als Treibmittel enthält.

2. Aerosollack nach Anspruch 1, dadurch gekennzeichnet, daß das N-Carboxybutylchitosan in einer Konzentration im Bereich zwischen 0,1 Gew.-% und 10 Gew.-% in bezug auf das Gesamtgewicht des Lacks vorhanden ist.

3. Aerosollack nach einem der Ansprüche 1 oder 2, dadurch gekennzeichnet, daß der wäßrige Träger aus Wasser oder einem Gemisch aus Wasser und einem organischen, kosmetisch annehmbaren Lösungsmittel besteht.

4. Aerosollack nach Anspruch 3, dadurch gekennzeichnet, daß das kosmetisch annehmbare organische Lösungsmittel als Ethanol, Isopropanol, Benzylalkohol, Phenylethylalkohol, Ethylenglykol, Propylenglykol oder als Gemisch aus diesen Lösungsmitteln vorliegt.

5. Aerosollack nach Anspruch 3 oder 4, dadurch gekennzeichnet, daß das Lösungsmittel in einem Mengenverhältnis von weniger als 50 Gew.-%, vorzugsweise in einem Bereich zwischen 5 Gew.-% und 30 Gew.-%, in bezug auf das Gesamtgewicht der Zusammensetzung vorhanden ist.

6. Aerosollack nach einem der vorstehenden Ansprüche, dadurch gekennzeichnet, daß er eine zweite filmbildende Substanz enthält.

7. Aerosollack nach einem der vorstehenden Ansprüche, dadurch gekennzeichnet, daß er zusätzlich noch einen Weichmacher in einem Mengenverhältnis von mindestens 10 Gew.-% in bezug auf das Gesamtgewicht der filmbildenden Substanzen enthält.

8. Aerosollack nach einem der vorstehenden Ansprüche, dadurch gekennzeichnet, daß der Dimethylether in einem Mengenverhältnis im Bereich zwischen 10 Gew.-% und 90 Gew.-%, vorzugsweise zwischen 20 Gew.-% und 40 Gew.-%, in bezug auf das Gesamtgewicht der Zusammensetzung vorhanden ist.

9. Aerosollack nach einem der vorstehenden Ansprüche, dadurch gekennzeichnet, daß er ein zweites Treibmittel enthält, das aus Chlorfluoralkanwasserstoffverbindungen, Fluoralkanwasserstoffverbindungen und nichthalogenierten Kohlenwasserstoffverbindungen sowie deren Gemischen ausgewählt ist.

10. Aerosollack nach einem der vorstehenden Ansprüche, dadurch gekennzeichnet, daß er zusätzlich noch einen Weichmacher, einen Duftstoff, ein Silikon, eine Sonnenfiltersubstanz, einen Farbstoff, ein Konservierungsmittel, einen Emulgator, ein Mittel gegen Schaumbildung, ein Vitamin, ein Protein, ein Hydratisierungsmittel oder ein Mittel gegen freie Radikale enthält.
